# EUROPEAN PATENT SPECIFICATION

(11) **EP 2 470 243 B1**
(45) Date of publication and mention of the grant of the patent: **02.10.2019**
(21) Application number: 10772991.5
(22) Date of filing: 26.08.2010
(51) Int. Cl.: A61M 5/24, A61M 5/28, A61M 5/34, A61M 5/315, A61M 5/31

(54) **ASSEMBLY KIT FOR PREPARING OF A PRE-FILLED SYRINGE**
BAUSATZ ZUR HERSTELLUNG EINER VORGEFÜLLTEN SPRITZE
KIT D'ASSEMBLAGE POUR PRÉPARER UNE SERINGUE PRÉ-REMPLIE

(30) Priority: 28.08.2009 EP 09011032
(43) Date of publication of application: 04.07.2012
(73) Proprietor: Sanofi-Aventis Deutschland GmbH, 65929 Frankfurt am Main (DE)
(72) Inventor: WAGNER, Daniel, 65926 Frankfurt am Main (DE); KÜHN, Bernd, 65926 Frankfurt am Main (DE); MÖCKEL, Jörn, 65926 Frankfurt am Main (DE)
(74) Representative: Weilnau, Carsten
(86) International application number: PCT/EP2010/062434
(87) International publication number: WO 2011/023738

(56) References cited:
- EP-A1- 0 328 504
- EP-A1- 2 062 607
- WO-A1-00/07646
- WO-A1-98/25660
- WO-A1-03/047667
- WO-A2-2008/131265
- GB-A- 2 308 302
- US-A- 2 870 766
- US-A- 3 967 759
- US-A- 5 067 947
- US-A- 5 509 903

## Description

### Field of the Invention

The present invention relates to a syringe assembly and in particular to a disposable pre-filled syringe assembly for administering of a liquid medicinal product to a patient, preferably by way of injection.

### Background and Prior Art

Numerous medicinal products like parenteral pharmaceuticals have to be administered by way of injection. For administration of local anaesthetics in particular dental anaesthetics, the active pharmaceutical ingredient is either provided in a multiple dose containing ampoule or vial, from which a required dose can be drawn up by a disposable syringe, which is subsequently used to administer the dose by injection. On the one hand, drawing up of a medicinal product from an ampoule or vial is considered as cumbersome and bears the risk of contamination of the sterile parenteral solution with microorganisms or particulates. On the other hand, medical staff is exposed to a non-negligible risk of injury when the tipped needle end is introduced into the multiple dosage container.

Alternatively, the active ingredient can be provided in a cartridge or carpule of substantially cylindrical shape, which is sealed by an axially displaceable bung. At its opposite end, the cartridge is typically sealed by means of a pierceable septum of elastomeric material. The septum is to be pierced by a needle, cannula or the like for expelling of the liquid product from the interior of the cartridge, in particular when the displaceable bung is subject to a distally directed displacement, that is, a displacement towards the septum.

Such cartridges filled with anaesthetics, in particular with dental anaesthetics, are usually inserted into and operably engaged with a rather large syringe rack, typically made of metal. The use of such syringe racks in combination with pre-filled cartridges quite often has a scary and threatening effect on the respective patient. The use of such over-dimensioned syringe racks therefore provokes a certain discomfort.

Moreover, use of such spacious syringe racks turned out to be particularly difficult for paediatric treatment. Also, multiple use of a syringe rack with different patients may increase the risk of cross infection.

Document WO 91/01152 A1 for instance discloses a pre-filled disposable syringe assembly for medicaments in injectable form. This syringe device includes a vial for medicaments that has a first open end and a sealed second end. On the first end, a plunger tip is positioned which seals that end and which, when moved to the other end, will force the contents of the vial out of the device. A finger grip is mounted adjacent to that first end.

The second end of the vial is sealed. A needle and hub means for slidably mounting a needle on the second end is provided and is in axial alignment with the sealed end so as to position the needle for access to the vial. Additionally, a plunger rod is provided having a first engagement means on one end for engaging the plunger tip. Further, a mounting device is provided for detachably mounting the plunger between the finger grip and the hub means.

An assembly kit with the features of the preamble of claim 1 is known from WO00/07646.

Such disposable pre-filled syringes are generally smaller in construction and shape compared to conventional syringe racks. However, pre-filled disposable syringes have to be individually adapted to and designed for the respective injectable medicinal product. Hence, in comparison with ampoules, vials or comparable bottles, costs for development and production for such disposable pre-filled syringes are comparatively high.

### Objects of the Invention

It is therefore an object of the present invention to provide a cost-efficient and universally applicable approach for injecting medicinal products, such like dental anaesthetics. The present invention further aims to provide a replacement of conventional syringe racks by disposable, more hygienic and less spacious injection means, being further usable with conventional cartridges or ampoules containing the medicinal product to be injected.

### Summary of the Invention

The invention provides an assembly kit for preparing of a pre-filled syringe with the features of claim 1, preferably of disposable type. The assembly kit comprises a gripping means to be attached to a proximal end section of a cartridge. The cartridge is of sleeve-like or cylindrical geometry and comprises an axially displaceable bung at its proximal end section for sealing of an interior volume of the cartridge. The interior volume of the cartridge is pre-filled with an injectable medicinal product, which can be expelled from the cartridge through a distally located outlet part, which is typically sealed by means of a septum being pierceable by a piercing element like a needle, a cannula or by comparable piercing or injection means.

The assembly kit further comprises a needle mount to be attached to the distal end section of the cartridge. The needle mount serves as a mount for a needle assembly to be coupled to the interior volume of the cartridge in a fluid-transferring way. Finally, the assembly kit comprises a plunger rod, which is connectable with the bung of the cartridge. By means of the plunger rod, a distally directed thrust can be applied to the bung, either manually or automatically, e.g. by means of an appropriate dispensing mechanism.

By means of the assembly kit, a pre-filled cartridge, such like a conventional carpule filled with dental anaesthetics can serve as a base for a pre-filled disposable syringe. Instead of arranging a cartridge in a rather spacious and over-dimensioned syringe rack, the at least three components of the assembly kit can be separately assembled at respective portions of the cartridge. In this way, a conventional cartridge or carpule can be easily and universally transformed into a disposable pre-filled syringe being prepared and adapted for injection of the respective medicinal product, such like dental anaesthetics.

Furthermore, the assembly kit comprises a protecting sleeve for at least partially embedding the cartridge therein. Since the cartridge typically comprises a barrel made of glass, the barrel or cartridge may become subject to breakage, e.g. when inadvertently dropped to the ground. The protecting sleeve now serves as a protection means and helps to prevent uncontrolled splintering of the cartridge in the event of breakage.

According to a preferred embodiment of the invention, the single components of the assembly kit, namely the gripping means, the needle mount and/or the plunger rod are made of plastic material, in particular of injection molded plastic material molded in a single or multi-component injection molding process.

By making use of plastic material for the assembly kit, manufacturing costs for the assembly kit can be kept in a remarkably low range and a conversion of a pre-filled cartridge into a disposable pre-filled syringe can be implemented in a cost-efficient way.

According to a further preferred embodiment, the gripping means and/or the needle mount are adapted to be frictionally and/or positively and/or adhesively engaged with respective peripheral or outer sections of the cartridge. Preferably, gripping means and/or needle mount are undetachably and preferably steadfastly engageable with the cartridge. By undetachably connecting the gripping means and/or the needle mount to the cartridge or carpule, a user is compelled to dispose the entire device when the content of the cartridge has been dispensed. By having the gripping means and/or the needle mount undetachably connected to the cartridge, e.g. a potential risk of cross infection due to multiple usage of the assembly kit can be reduced.

In a further preferred embodiment, the plunger rod is adapted to become frictionally and/or positively and/or adhesively engaged with the bung. For this purpose, a distally located end section of the plunger rod and a respective proximally directed end face of the bung may comprise mutually corresponding interlocking means, such like a snap fit or a threaded engagement.

Furthermore and according to another preferred embodiment, the plunger rod and/or the gripping means comprise a ring portion adapted to receive a user's finger. By way of having a closed or open ring portion, the overall handling of the assembly kit and the pre-filled syringe is facilitated. Hence, a user may grip the gripping means with his index finger while making use of his thumb to exert distally directed pressure onto the plunger rod or to retract the plunger stopper to allow for aspiration in case of non-intravascular injections.

In a further embodiment, the injection needle or cannula is pre-assembled to the needle mount before said needle mount itself is attached to the cartridge. Alternatively, the needle mount is attached to the cartridge prior to a fluid-transferring coupling of injection needle and cartridge.

The protecting sleeve serves to provide a splinter shield particularly in the event that the material of the cartridge becomes subject to breakage, which might be the case when the cartridge is made of glass. The protecting sleeve can be designed so as to be attached exclusively to the outer circumference of the cartridge or its barrel. Hence, the protecting sleeve preferably acts as a sheath at least partially encasing the cartridge.

The protecting sleeve is adhesively attached almost across the entire outer surface of the cartridge or its barrel.

In an example, that does not make part of the present invention, it is even conceivable, that the protecting sleeve is designed as proximal extension of the needle mount. This way, the needle mount comprises a sleeve-like receptacle, which is adapted to at least partially house the cartridge. Such sleeve-like receptacle on the one hand provides a protection of the cartridge and on the other hand serves as splinter shield, adapted to prevent uncontrolled spreading of glass splinters, in case the cartridge is subject to breakage.

In another example, the protecting sleeve may comprise a flexible or nonelastic material. It may be positively engaged and/or attached to the cartridge.

According to the invention, the protecting sleeve comprises a foil adhesively attached to the outer circumference of the cartridge. The protecting sleeve may even consist of the foil.

In particular when the foil is substantially transparent, the foil or the protective sleeve is hardly visible or recognizable by the end user. Also, the overall design and appearance of the cartridge remains substantially unaltered. Since the foil is particularly flexible, breakage of the cartridge leading to a number of splinters may no longer be harmful to the end user, because in the event of breakage, the splintered parts of the cartridge remain adhesively attached to the foil.

Depending on the configuration of cartridge and the various components of the assembly kit, attachment of gripping means, needle mount and plunger rod to respective portions of the cartridge may not necessarily have to be conducted under aseptic or sterile conditions. If for instance the needle mount is pre-assembled with an injection needle or the like, assembling of needle mount to the distal end section of the cartridge will be preferably conducted under at least sterile or aseptic conditions.

According to another example refers to a cartridge for a drug delivery device, wherein the cartridge comprises a substantially cylindrical barrel made of glass and further has an axially displaceable bung disposed therein for sealing an interior volume of the barrel. Furthermore, the cartridge comprises a protecting foil or a respective meshed grid at least partially adhesively attached to the barrel for providing a splinter shield in the event of breakage of the barrel.

In a further aspect, the invention provides a method of preparing of a pre-filled syringe assembly according to claim 8 on the basis of a pre-filled cartridge. The cartridge contains an injectable medicinal product and is sealed with an axially displaceable bung. For preparing or transforming the pre-filled cartridge into a pre-filled syringe, at least a gripping means is attached to a proximal end section of the cartridge, a needle mount is attached to a distal end section of the cartridge, which provides a mount for a needle assembly to be coupled to the interior volume of the cartridge in a fluid transferring way. The method further comprises the step of attaching of a plunger rod to the bung of the cartridge in order to provide application of distally directed thrust to the bung, in particular for expelling the medicinal product from the cartridge. Moreover, the cartridge is at least partially embedded in a protecting sleeve. This final step of preparing the cartridge with a protecting sleeve to be attached thereto may already be conducted in the course of manufacturing of the cartridge.

By way of assembling at least three components of an assembly kit to a pre-filled cartridge, said cartridge or carpule can be transformed or converted into a pre-filled syringe, which is adapted for administering the medicinal product by way of injection.

By making use of a pre-filled cartridge as an integral or base component of a syringe, rather time and cost-intensive compatibility- and stability investigation can be saved, that would otherwise be necessary for the development of an comparable disposable pre-filled syringe. Hence, for the present invention existing cartridges and carpules do not have to be modified but can be directly used in connection with the assembly kit.

According to a further preferred embodiment, a needle assembly, typically comprising a tipped cannula or needle is attached to the needle mount after securing of the needle mount to the cartridge. Since the needle mount itself does not penetrate the cartridge's septum, preparation of the pre-filled syringe can even be conducted in non-sterile or non-aseptic environment. If the needle assembly is to be attached to the needle mount after conversion or transformation of the cartridge, only said needle assembly has to be attached, preferably under sterile or aseptic conditions.

In a further independent aspect, the invention refers to the use of a pre-filled cartridge, in combination with an assembly kit for preparation of a pre-filled syringe assembly, according to claim 10. The cartridge comprises an axially displaceable bung for sealing an interior volume of the cartridge being pre-filled with an injectable medicinal product. The cartridge itself serves as a base unit, which is adapted for attaching of at least a gripping means, a needle mount, a plunger rod and/or a protecting sleeve thereto for preparing a pre-filled syringe assembly, hence for converting or transforming the pre-filled cartridge into a preferably disposable pre-filled and ready-to-use syringe assembly.

It will be apparent to those skilled in the pertinent art that various modifications and variations can be made to the present invention as defined by the claims. Further, it is to be noted, that any reference signs used in the appended claims are not to be construed as limiting the scope of the present invention.

### Brief Description of the Drawings

Without limitation, the present invention will be explained in greater detail below in connection with preferred embodiments and with reference to the drawings in which:
- Figure 1: schematically illustrates a three-dimensional view of a pre-filled cartridge,
- Figure 2: schematically shows an assembly kit attached to the cartridge, that is not part of the invention,
- Figure 3: illustrates an assembly kit according to the invention,
- Figure 4: separately illustrates the gripping means,
- Figure 5: shows a snap-fit coupling of plunger rod and bung,
- Figure 6: illustrates a threaded engagement of plunger rod and bung and
- Figure 7: is illustrative of a clamped mutual engagement of plunger rod and bung.

### Detailed Description

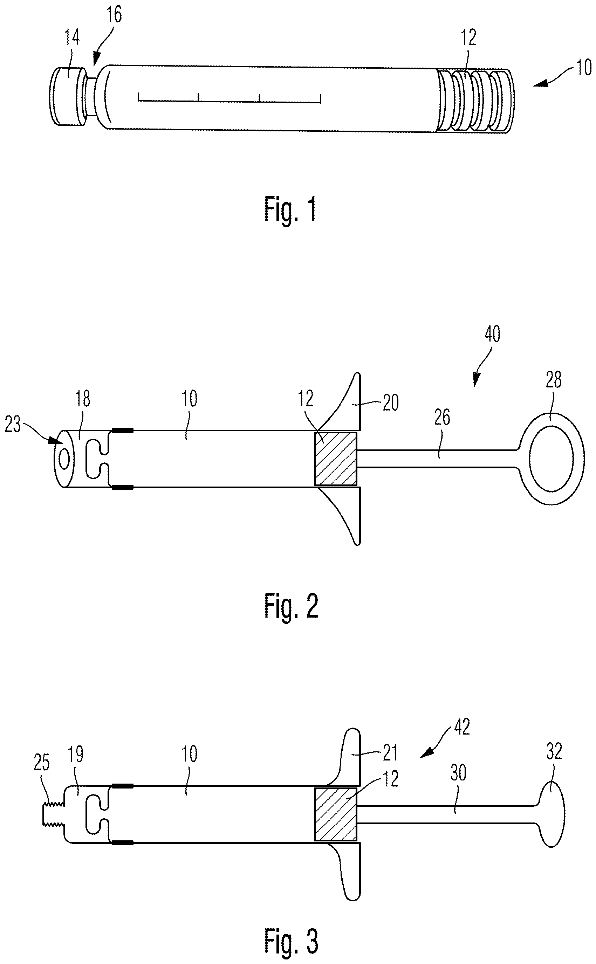

The cartridge 10 as illustrated in Figure 1 comprises a substantially cylindrical or sleeve-like shape. At its left and distal end section, the cartridge or carpule 10 comprises a neck portion 16, which forms an outlet port through which the medicinal product contained in said cartridge 10 is to be expelled. Further but not illustrated, the left and distal end section of the cartridge 10 is sealed by a septum, which is pierceable by a piercing element, such like an injection needle or cannula. The septum is fixed to the outlet part by means of a metallic cap 14.

The illustrated carpule 10 is commercially available for various parenteral medications, in a preferred example it contains dental anaesthetics. For injection of such dental anaesthetics, the carpule 10 is conventionally to be coupled with and to be mounted in a syringe rack, wherein the bung 12 is pushed to the left, hence in distal direction for expelling of a required dose of the anaesthetic pharmaceutical.

According to the present invention, the pre-filled carpule 10 is transformed and converted into a pre-filled syringe. For this purpose, a needle mount 18 is attached to the neck portion 16 of the carpule 10 whereas a gripping means 20 is attached near the opposite, proximal end section of the cartridge 10. Finally, also a plunger rod 26 is attached to the bung 12 allowing for exertion of distally directed pressure to the bung 12 for expelling of the liquid medicinal product contained in the carpule 10.

The plunger rod 26 optionally can comprise a finger ring 28 at its proximal end section. The ring 28 is particularly adapted to receive a thumb of a user while the gripping means 20 with its radially protruding ring portions 24 is gripped by means of index finger and middle finger, respectively.

By attaching the illustrated components 18, 20, 26 of said assembly kit to the cartridge 10, the cartridge 10 itself transforms into a pre-filled syringe 40.

The pre-filled syringe 42 as illustrated in Figure 3 comprises a slightly differently configured assembly kit. Whereas the needle mount 18 of the example of figure 2 comprises a connector 23, e.g in form of a Luer-Lock connector or the like, the needle mount according to the invention 19 of pre-filled syringe 42 comprises a threaded socket 25, adapted to be threadedly engaged with a correspondingly threaded but not further illustrated needle holder.

The gripping means 21 of the pre-filled syringe 42 only differs in shape and geometry from the gripping means 20 of the syringe 40. However, gripping means 20 and 21 effectively operate in the same way. The plunger 30 of pre-filled syringe 42 comprises a radially extending flange-like button 32 providing an appropriately sized surface for receiving of a user's thumb.

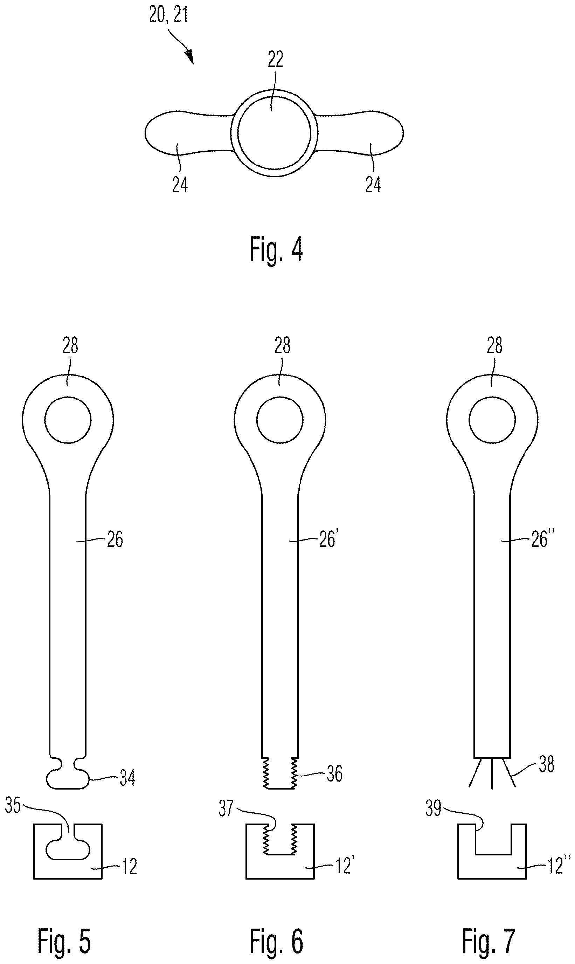

As further illustrated in Figure 4, both gripping means 20, 21 comprise an aperture or through opening 22, which is adapted to receive the substantially cylindrical body of the cartridge 10. Preferably, the cartridge 10 comprises a radially outwardly protruding flange, which axially abuts with the circumference of said aperture 22. The wing portions 24 extending in diametrically opposing radial directions may comprise an ergonomically shaped surface in order to support a secure and rather slip-free gripping of the gripping means 20, 21 and of the entire pre-filled syringe 40, 42.

Even though not further illustrated, the gripping means 20, 21 and the needle mount 18, 19 may be connected by a sleeve-like cover that can be made of a fixed or flexible material or a foil, which is preferably transparent. This sleeve-like cover is particularly of advantage, when the housing of the cartridge is made of breakable material, such like glass. According to the invention, there is a foil embedding the cartridge 10, in case of cartridge damaging, a risk of injury, that may arise from splinters of glass, can be reduced to a minimum.

In Figure 5, a possible attachment of plunger rod 26 and bung 12 is illustrated. Here, the lower, distal end section 34 of the plunger rod 26 comprises a radially constricted connecting end 34, being insertable in a correspondingly shaped receptacle 35 of the bung 12, thereby establishing a positive interlocking.

In the embodiment as illustrated in Figure 6, the plunger rod 26' comprises a threaded connecting end 36 designed for establishing a threaded engagement with the correspondingly threaded receptacle 37 of the bung 12'.

Alternatively, as illustrated in Figure 7 the distal connecting end of the plunger rod 26" comprises protruding spikes or prongs 38 adapted to penetrate the bung 12" when positioned inside respective receptacle 39.

### List of Reference Numerals

- 10: cartridge
- 12: bung
- 14: cap
- 16: neck portion
- 18: needle mount
- 19: needle mount
- 20: gripping means
- 21: gripping means
- 22: aperture
- 23: connector
- 24: ring portion
- 25: threaded socket
- 26: plunger rod
- 28: finger ring
- 30: plunger rod
- 32: button portion
- 34: connecting end
- 35: receptacle
- 36: connecting end
- 37: receptacle
- 38: prong
- 39: receptacle
- 40: syringe assembly
- 42: syringe assembly

## Claims

1. An assembly kit for preparing of a pre-filled syringe, comprising:
- a gripping means (20, 21) to be attached to a proximal end section of a cartridge (10), which comprises an axially displaceable bung (12) for sealing of an interior volume of the cartridge (10) being pre-filled with an injectable medicinal product, wherein the cartridge (10) comprises a distal end section sealed by a septum, wherein the septum is pierceable by an injection needle or cannula of a needle assembly,
- a plunger rod (26; 30) connectable with the bung (12) of the cartridge (10) for applying distally directed thrust to the bung (12), and
wherein the cartridge (10) is at least partially embedded in a protecting sleeve, wherein
- a needle mount (19) undetachably and steadfastly attachable to the distal end section of the cartridge (10) and providing a mount for the needle assembly to be coupled to the interior volume of the cartridge (10) in a fluid-transferring way, **characterised in that** the needle mount (19) comprises a threaded socket (25) with an outer thread to be threadedly engaged with the correspondingly threaded needle assembly configured as a needle holder and
- wherein the protecting sleeve comprises a foil adhesively attached almost across the entire outer surface of the cartridge (10).

2. The assembly kit according to claim 1, wherein the gripping means (20, 21), the needle mount (19) and/or the plunger rod (26, 30) comprise injection molded plastic material.

3. The assembly kit according to any one of the preceding claims, wherein the gripping means (20, 21) and/or the needle mount (19) are adapted to be frictionally and/or positively and/or adhesively engaged with respective peripheral sections of the cartridge (10).

4. The assembly kit according to any one of the preceding claims, wherein the gripping means (20, 21) and/or the needle mount (19) are undetachably engageable with the cartridge (10).

5. The assembly kit according to any one of the preceding claims, wherein the plunger rod (26; 30) is adapted to become frictionally and/or positively and/or adhesively engaged with the bung (12).

6. The assembly kit according to any one of the preceding claims, wherein the plunger rod (26; 30) and/or the gripping means (20, 21) comprise a ring portion (28) adapted to receive a user's finger.

7. A pre-filled syringe comprising:
- a cartridge (10) pre-filled with an injectable medicinal product and comprising a proximal end section, an axially displaceable bung (12) for sealing of an interior volume of the cartridge and a distal end section sealed by a septum pierceable by an injection needle or cannula of a needle assembly,
- an assembly kit according to any one of the preceding claims.

8. A method of preparing of a pre-filled syringe assembly starting from a pre-filled cartridge (10), which contains an injectable medicinal product and which is sealed with an axially displaceable bung (12) and which comprises a distal end section sealed by a septum, wherein the septum is pierceable by an injection needle or cannula of a needle assembly, the method of preparing the pre-filled syringe comprising the steps of:
- attaching of a gripping means (20, 21) to a proximal end section of the cartridge (10)
- undetachably and steadfastly attaching of a needle mount (19) to the distal end section of the cartridge (10), wherein the needle mount (19) provides a mount for a needle assembly to be coupled to the interior volume of the cartridge (10) in a fluid-transferring way, wherein the needle mount (19) comprises a threaded socket (25) with an outer thread to be threadedly engaged with the correspondingly threaded needle assembly configured as a needle holder,
- attaching of a plunger rod (26; 30) to the bung (12) of the cartridge (10) for applying distally directed thrust to the bung (12), and
- adhesively attaching of a foil almost across the entire outer surface of the cartridge (10).

9. The method according to claim 8, wherein the needle assembly is attached to the needle mount (19) after securing of the needle mount (19) to the cartridge (10).

10. Use of a pre-filled cartridge (10) for preparation of a pre-filled syringe according to claim 7, wherein the cartridge (10) comprises an axially displaceable bung (12) sealing an interior volume of the cartridge (10) being pre-filled with an injectable medicinal product, wherein the cartridge (10) comprises a distal end section sealed by a septum, wherein the septum is pierceable by an injection needle or cannula of a needle assembly and wherein the cartridge (10) serves as a base unit adapted for attaching of at least a gripping means (20, 21), a needle mount (19) a plunger rod (26; 30) and a protecting sleeve thereto for the purpose of preparing a pre-filled syringe assembly.

## Patentansprüche

1. Bausatz zum Bereitstellen einer vorgefüllten Spritze, aufweisend:
- ein an einem proximalen Endabschnitt einer Kartusche (10) anzubringendes Greifmittel (20, 21), das einen axial verschiebbaren Pfropfen (12) zum Abdichten eines mit einem injizierbaren medizinischen Produkt vorgefüllten inneren Volumens der Kartusche (10) aufweist, wobei die Kartusche (10) einen mit einem Septum abgedichteten distalen Endabschnitt aufweist, wobei das Septum mittels einer Injektionsnadel oder Kanüle einer Nadelanordnung durchstechbar ist,
- eine mit dem Pfropfen (12) der Kartusche (10) verbindbare Kolbenstange (26; 30) um einen distal ausgerichteten Druck auf den Pfropfen (12) auszuüben, und
wobei die Kartusche (10) zumindest teilweise in eine Schutzhülse eingebettet ist, wobei
- eine Nadelhalterung (19) untrennbar und fest an dem distalen Endabschnitt der Kartusche (10) anbringbar ist und eine Halterung für die Nadelanordnung bereitstellt, die auf eine fluidtransferierende Weise an das innere Volumen der Kartusche (10) zu koppeln ist, **dadurch gekennzeichnet, dass** die Nadelhalterung (19) eine Gewindefassung (25) mit einem Außengewinde aufweist, um mit der mit einem entsprechenden Gewinde versehenen Nadelanordnung, die als ein Nadelhalter ausgestaltet ist, in Gewindeeingriff zu gelangen, und
- wobei die Schutzhülse eine Folie aufweist, die fast über die gesamte Außenfläche der Kartusche (10) aufgeklebt ist.

2. Bausatz nach Anspruch 1, wobei das Greifmittel (20, 21), die Nadelhalterung (19) und/oder die Kolbenstange (26, 30) spritzgegossenes Kunststoffmaterial aufweisen.

3. Bausatz nach einem der vorhergehenden Ansprüche, wobei das Greifmittel (20, 21) und/oder die Nadelhalterung (19) dazu ausgeführt sind, mit jeweiligen Umfangsabschnitten der Kartusche (10) in Reib- und/oder Formschluss- und/oder Klebeeingriff zu gelangen.

4. Bausatz nach einem der vorhergehenden Ansprüche, wobei das Greifmittel (20, 21) und/oder die Nadelhalterung (19) untrennbar mit der Kartusche (10) in Eingriff bringbar sind.

5. Bausatz nach einem der vorhergehenden Ansprüche, wobei die Kolbenstange (26; 30) dazu ausgeführt ist, mit dem Pfropfen (12) in Reib- und/oder Formschluss- und/oder Klebeeingriff zu gelangen.

6. Bausatz nach einem der vorhergehenden Ansprüche, wobei die Kolbenstange (26; 30) und/oder das Greifmittel (20, 21) einen Ringabschnitt (28) aufweisen, der zur Aufnahme eines Benutzerfingers ausgeführt ist.

7. Vorgefüllte Spritze, aufweisend:
- eine Kartusche (10), die mit einem injizierbaren medizinischen Produkt vorgefüllt ist und einen proximalen Endabschnitt, einen axial verschiebbaren Pfropfen (12) zum Abdichten eines inneren Volumens der Kartusche und einen distalen Endabschnitt aufweist, der mit einem Septum abgedichtet ist, das mittels einer Injektionsnadel oder Kanüle einer Nadelanordnung durchstechbar ist,
- einen Bausatz nach einem der vorhergehenden Ansprüche.

8. Verfahren zum Bereitstellen einer vorgefüllten Spritzenanordnung, ausgehend von einer vorgefüllten Kartusche (10), die ein injizierbares medizinisches Produkt enthält und die mit einem axial verschiebbaren Pfropfen (12) abgedichtet ist und die einen mit einem Septum abgedichteten distalen Endabschnitt aufweist, wobei das Septum mittels einer Injektionsnadel oder Kanüle einer Nadelanordnung durchstechbar ist, wobei das Verfahren zum Bereitstellen der vorgefüllten Spritze die folgenden Schritte aufweist:
- Anbringen eines Greifmittels (20, 21) an einem proximalen Endabschnitt der Kartusche (10),
- untrennbares und festes Anbringen einer Nadelhalterung (19) an dem distalen Endabschnitt der Kartusche (10), wobei die Nadelhalterung (19) eine Halterung für eine auf eine fluidtransferierende Weise an das innere Volumen der Kartusche (10) zu koppelnde Nadelanordnung bereitstellt, wobei die Nadelhalterung (19) eine Gewindefassung (25) mit einem Außengewinde aufweist, um mit der mit einem entsprechenden Gewinde versehenen Nadelanordnung, die als ein Nadelhalter ausgestaltet ist, in Gewindeeingriff zu gelangen,
- Anbringen einer Kolbenstange (26; 30) an dem Pfropfen (12) der Kartusche (10) zur Beaufschlagung des Pfropfens (12) mit distal ausgerichtetem Druck und
- Ankleben einer Folie fast über die gesamte Außenfläche der Kartusche (10).

9. Verfahren nach Anspruch 8, wobei die Nadelanordnung nach dem Befestigen der Nadelhalterung (19) an der Kartusche (10) an der Nadelhalterung (19) angebracht wird.

10. Verwendung einer vorgefüllten Kartusche (10) zur Bereitstellung einer vorgefüllten Spritze nach Anspruch 7, wobei die Kartusche (10) einen axial verschiebbaren Pfropfen (12) aufweist, der ein mit einem injizierbaren medizinischen Produkt vorgefülltes inneres Volumen der Kartusche (10) abdichtet, wobei die Kartusche (10) einen mit einem Septum abgedichteten distalen Endabschnitt aufweist, wobei das Septum mittels einer Injektionsnadel oder Kanüle einer Nadelanordnung durchstechbar ist und wobei die Kartusche (10) als eine Basiseinheit dient, die zwecks Bereitstellens einer vorgefüllten Spritzenanordnung zum Anbringen zumindest eines Greifmittels (20, 21), einer Nadelhalterung (19), einer Kolbenstange (26; 30) und einer Schutzhülse daran ausgestaltet ist.

## Revendications

1. Kit d'assemblage pour préparer une seringue pré-remplie, comprenant :
- un moyen de préhension (20, 21) destiné à être attaché à une section d'extrémité proximale d'une cartouche (10), qui comprend un bouchon déplaçable axialement (12) pour sceller un volume intérieur de la cartouche (10) lorsqu'elle est pré-remplie avec un produit médicinal injectable, la cartouche (10) comprenant une section d'extrémité distale scellée par un septum, le septum pouvant être percé par une aiguille d'injection ou une canule d'un ensemble d'aiguille,
- une tige de plongeur (26 ; 30) pouvant être connectée au bouchon (12) de la cartouche (10) pour appliquer une poussée orientée distalement sur le bouchon (12), et
la cartouche (10) étant au moins en partie enveloppée dans un manchon protecteur,
dans lequel
- un support d'aiguille (19) peut être attaché de manière indétachable et solide à la section d'extrémité distale de la cartouche (10) et fournit un support permettant à l'ensemble d'aiguille d'être accouplé au volume intérieur de la cartouche (10) de manière à pouvoir transférer un fluide,
**caractérisé en ce que**
le support d'aiguille (19) comprend une douille filetée (25) avec un filetage extérieur destiné à venir en prise par filetage avec l'ensemble d'aiguille pourvu d'un filetage correspondant, configuré sous forme de moyen de retenue d'aiguille et
- le manchon protecteur comprenant un film attaché de manière adhésive pratiquement sur toute la surface extérieure de la cartouche (10) .

2. Kit d'assemblage selon la revendication 1, dans lequel le moyen de préhension (20, 21), le support d'aiguille (19) et/ou la tige de plongeur (26, 30) comprennent un matériau en plastique moulé par injection.

3. Kit d'assemblage selon l'une quelconque des revendications précédentes, dans lequel le moyen de préhension (20, 21) et/ou le support d'aiguille (19) sont prévus pour être mis en prise par friction et/ou par engagement positif et/ou par adhésion avec des sections périphériques respectives de la cartouche (10).

4. Kit d'assemblage selon l'une quelconque des revendications précédentes, dans lequel le moyen de préhension (20, 21) et/ou le support d'aiguille (19) peuvent être mis en prise de manière indétachable avec la cartouche (10).

5. Kit d'assemblage selon l'une quelconque des revendications précédentes, dans lequel la tige de plongeur (26 ; 30) est prévue pour venir en prise par friction et/ou par engagement positif et/ou par adhésion avec le bouchon (12).

6. Kit d'assemblage selon l'une quelconque des revendications précédentes, dans lequel la tige de plongeur (26 ; 30) et/ou le moyen de préhension (20, 21) comprennent une partie en forme de bague (28) prévue pour recevoir le doigt d'un utilisateur.

7. Seringue pré-remplie, comprenant :
- une cartouche (10) pré-remplie avec un produit médicinal injectable et comprenant une section d'extrémité proximale, et un bouchon déplaçable axialement (12) pour sceller un volume intérieur de la cartouche et une section d'extrémité distale scellée par un septum pouvant être percé par une aiguille d'injection ou une canule d'un ensemble d'aiguille,
- un kit d'assemblage selon l'une quelconque des revendications précédentes.

8. Procédé de préparation d'un ensemble de seringue pré-remplie à partir d'une cartouche pré-remplie (10), qui contient un produit médicinal injectable et qui est scellée avec un bouchon déplaçable axialement (12) et qui comprend une section d'extrémité distale scellée par un septum, le septum pouvant être percé par une aiguille d'injection ou une canule d'un ensemble d'aiguille, le procédé de préparation de la seringue pré-remplie comprenant les étapes consistant à :
- attacher un moyen de préhension (20, 21) à une section d'extrémité proximale de la cartouche (10),
- attacher de manière indétachable et solide un support d'aiguille (19) à la section d'extrémité distale de la cartouche (10), le support d'aiguille (19) fournissant un support permettant à l'ensemble d'aiguille d'être accouplé au volume intérieur de la cartouche (10) de manière à pouvoir transférer un fluide, le support d'aiguille (19) comprenant une douille filetée (25) avec un filetage extérieur destiné à venir en prise par filetage avec l'ensemble d'aiguille pourvu d'un filetage correspondant, configuré sous forme de moyen de retenue d'aiguille,
- attacher une tige de plongeur (26 ; 30) au bouchon (12) de la cartouche (10) pour appliquer une poussée orientée distalement sur le bouchon (12), et
- attacher un film de manière adhésive pratiquement sur toute la surface extérieure de la cartouche (10).

9. Procédé selon la revendication 8, dans lequel l'ensemble d'aiguille est attaché au support d'aiguille (19) une fois que le support d'aiguille (19) est fixé à la cartouche (10).

10. Utilisation d'une cartouche pré-remplie (10) pour la préparation d'une seringue pré-remplie selon la revendication 7, dans laquelle la cartouche (10) comprend un bouchon déplaçable axialement (12) scellant un volume intérieur de la cartouche (10) lorsqu'elle est pré-remplie avec un produit médicinal injectable, la cartouche (10) comprend une section d'extrémité distale scellée par un septum, le septum peut être percé par une aiguille d'injection ou une canule d'un ensemble d'aiguille et la cartouche (10) sert d'unité de base prévue pour attacher au moins un moyen de préhension (20, 21), un support d'aiguille (19), une tige de plongeur (26 ; 30) et un manchon protecteur à celle-ci, afin de préparer un ensemble de seringue pré-remplie.
